# EUROPÄISCHE PATENTANMELDUNG

(11) **EP 2 876 103 A1**
(43) Veröffentlichungstag der Anmeldung: **27.05.2015**
(21) Anmeldenummer: 13193711.2
(22) Anmeldetag: 20.11.2013
(51) Int. Cl.: C07C 231/02, C07C 235/20, C11D 1/52

(54) **Teikristalline Glucamid-Zusammensetzungen und Verfahren zu deren Herstellung**

(71) Anmelder: Clariant International Ltd., 4132 Muttenz (CH)
(72) Erfinder: Dahms, Gerd, 47138 Duisburg (DE); Klug, Peter, 63762 Großostheim (DE)
(74) Vertreter: Michalski Hüttermann & Partner Patentanwälte mbB

(57) **Zusammenfassung**

Die Erfindung betrifft eine teilkristalline N-Alkyl-N-Acylglucamin-Zusammensetzung umfassend mindestens zwei unterschiedliche Acylglucamine mit Acylresten ausgewählt aus der Gruppe der gesättigten oder ungesättigten C6-C22 Acyle, wobei die Zusammensetzung bei Raumtemperatur eine teilkristalline Struktur mit mindestens jeweils einen signifikanten Röntgenreflex in den 2Theta-Bereichen ≥ 13,5° bis ≤ 15,1° und ≥ 15,5° bis ≤ 17,4° aufweist, wobei die Röntgenreflexe durch ein Pulver-Diffraktogramm in Bragg-Brentano-Geometrie erhalten werden.

## Beschreibung

Die Erfindung betrifft eine teilkristalline N-Alkyl-N-Acylglucamin-Zusammensetzung umfassend mindestens zwei unterschiedliche Acylglucamine mit Acylresten ausgewählt aus der Gruppe der gesättigten oder ungesättigten C6-C22 Acyle, wobei die Zusammensetzung bei Raumtemperatur eine teilkristalline Struktur mit mindestens jeweils einen signifikanten Röntgenreflex in den 2Theta-Bereichen ≥ 13,5° bis ≤ 15,1° und ≥ 15,5° bis ≤ 17,4° aufweist, wobei die Röntgenreflexe durch ein Pulver-Diffraktogramm in Bragg-Brentano-Geometrie erhalten werden.

Die Qualitätsanforderungen an moderne Wasch- und Reinigungsmittel sind in den letzen Jahrzehnten deutlich vielschichtiger geworden. Neben einer guten Reinigungsleistung erwartet der Verbraucher heutzutage eine gute (Umwelt)Verträglichkeit, maßgeschneiderte Handhabbarkeit, leichte biologische Abbaubarkeit sowie die Verwendung nachhaltiger Ressourcen. Ein wesentlicher Punkt zur Erfüllung dieses Anforderungsprofils liegt dabei in der Bereitstellung waschaktiver Substanzen, in den meisten Fällen amphiphiler Moleküle, welche im Rahmen der gewählten Formulierung und Anwendungssituation in wichtigen Handhabungsparametern wie Kaltlöse-, Auflöse-, Fließ- und Verdünnungsverhalten sowie Schmutztragefähigkeit und Verdickbarkeit sehr gute Eigenschaften aufweisen sollen. Diese Gemengelage stellt gerade im Bereich waschaktiver Substanzen eine große Herausforderung dar, da die spezielle Wirkweise amphiphiler Substanzen sich im Gegensatz zu den Interaktionen anderer Stoffklassen, in den allermeisten Fällen nicht adäquat durch einfache Einzelmolekül-Einzelmolekül-Beziehungen beschreiben lässt. In der Regel basieren die gängigsten industriellen Anwendungsbereiche dieser Substanzen, wie beispielsweise Entschäumer, (W/O)/(O/W)-Emulgatoren, Netzmittel, Tenside oder Lösungsvermittler, auf einem komplexen Zusammenspiel sowohl der Amphiphile untereinander wie auch dazu in Relation stehender, zusätzlicher Wechselwirkung der Amphiphile mit der potentiellen Zielmatrix. Das Verständnis beider Wechselwirkungsklassen, der Amphiphil-Amphiphil und Amphiphil-Zielmatrix, ist dabei gleichermaßen wichtig, um für die jeweilige Einsatzsituation maßgeschneiderte Eigenschaften bereitstellen zu können.

In der Substanzklasse der nichtionischen Tenside haben sich die N-Alkyl-N-Acylglucamine (Glucamide) als besonders geeignet erwiesen, obiges Anforderungsprofil erfüllen zu können. Diese Substanzklasse ist dermatologisch hoch akzeptabel, biologisch gut abbaubar, zeigt eine ausgesprochen gute Reinigungswirkung und lässt sich aus nachwachsenden Rohstoffen bereitstellen. Aus diesem Grund findet sich in der Patentliteratur eine Vielzahl an Dokumenten, welche unterschiedliche Strategien zur Herstellung dieser Moleküle anführen.

So beschreibt beispielsweise die US 5,194,639 A ein Verfahren zur Herstellung aufgereinigter, im Wesentlichen nicht zyklischer Polyhydroxy-Fettsäureamide, in welchem ein Fettsäureester und ein N-Alkyl-Polyhydroxyamin mit einem oder mehreren hydroxy- oder alkoxylierten Lösungsmitteln in Gegenwart eines basischen Katalysators reagieren. Der besagte Prozess weist folgende Parameter auf: a) der basische Katalysator ist ein Alkoxid, b) der Prozess wird in einem Temperaturbereich von ca. 25 °C - 130 °C durchgeführt, das Gewichtsverhältnis zwischen Fettsäureester und N-Alkyl-Polyhydroxyamin beträgt mindestens ca. 1:1, besagter Fettsäureester ist ein C12-C20 Fettsäureester und e) besagtes Lösungsmittel ist ein C1-C4 Alkohol, Ethylenglykol, Propylenglykol, Glycerin, alkoxylierter Alkohol oder Mischungen derselben.

Einen weiteren Weg zu Herstellung linearer Glucamid-Tenside kann man der US 5,338,486 entnehmen. In dieser wird das Glucamid durch Reaktion eines N-Alkyl-Glucamins, beispielsweise N-Methyl-Glucamin, eines Fettsäureesters, beispielweise Kokosnussmethylester unter Gegenwart eines hochkonzentrierten Katalysators ausgewählt aus Trilitiumphosphat, Trinatriumphosphat, Trikaliumphosphat, Tetranatriumpyrophosphat, Tetrakaliumpyrophosphat, Pentanatriumtripolyphosphat, Pentakaliumtripolyphosphat, Lithiumcarbonat, Natriumcarbonat, Kaliumcarbonat, Dinatriumtartrat, Dikaliumtartrat, Natriumkaliumtartrat, Trinatriumcitrat, Trikaliumcitrat, basische Natriumsilikate, basische Kaliumsilikate, basische Barium-Alumosilikate, basische Kalium-Alumosilikate und Mischungen derselben, erhalten.

Desweiteren findet sich in der US 5,380,891 A ein mögliche Ausgestaltung zur Darstellung linearer N-Alkyl-Glucamide. Hierin wird ein N-Alkyl-Glucamin, beispielsweise N-Methylglucamin, ein Fettsäureester, beispielweise Kokosnussöl, in Gegenwart eines Phasentransfer-Agens, ein nicht-ionischer oberflächenaktiver Stoff, bevorzugt ein schon vorgefertigtes Produkt des Prozesses, bevorzugt in Gegenwart eines alkalischen Katalysators zur Reaktion gebracht.

In der US 5,723,673 A wird hingegen ein Weg zur Darstellung von N-Alkyl-PolyhydroxyAmin-Amiden mit guter Farbe beschrieben. Es wird ein N-Alkyl-Polyhydroxyalkyl-Amin, wie N-Methylglucamin, mit einer Gardner-Farbzahl kleiner 1 mit einer Quelle von Fettsäureacylgruppen wie Methylestern, Anhydriden und/oder Fettsäuren mit einer Transmission von größer als 98% bei 460 nm in organischen Hydroxy-Lösungen wie Methanol zur Reaktion gebracht. Die N-Alkyl-Polyhydroxyamine können durch Kristallisation und oder durch Bleichen zum Erhalt einer verbesserten Farbe aufgereinigt werden. Zur Verringerung des Anteils zyklischer Reaktionsprodukte kann die Reaktion vorzugsweise bei tiefen Temperaturen, in kurzen Zeitperioden und mit einem geringen Katalysatoranteil durchgeführt werden. Das erhaltene Amidprodukt kann desweiteren mittels Anionen- und Kationen-Austauschersäulen unter Entfernung von Seifen und Aminen gereinigt werden.

Die WO 9 410 130 A1 beschriebt andererseits die Herstellung von N-Alkanoylpolyhydroxyalkylaminen Z-NR¹-CO-R² wobei Z für den Polyhydroxyalkylrest eines Mono- oder Oligosaccharides steht, R¹ Wasserstoff oder C₁-C₈-Alkyl und R² C₁-C₂₁-Alkyl bezeichnet. Die Herstellung erfolgt durch Umsetzung von Polyhydroxyalkylaminen Z-NH-R¹, mit Carbonsäurealkylestern R³-O-CO-R², wobei R³ einen C₁- bis C₄-Alkylrest bedeutet, in Gegenwart eines basischen Katalysators. Hierzu wird (a) die Gesamtmenge des Esters vorlegt, auf die Umsetzungstemperatur erwärmt und das Amin in Form einer Schmelze unter Fortschreiten der Umsetzung zudosiert, wobei gebildeter Alkohol R³OH fortlaufend abdestilliert wird, (b) die Umsetzung bei einer Temperatur von 70 bis 110°C durchgeführt und (c) die Umsetzung in Abwesenheit organischer Lösungsmitteln vorgenommen.

Es hat sich allerdings herausgestellt, dass die Verarbeitungs- und Wascheigenschaften der N-Alkyl-N-Acylglucamine dann besonders vorteilhaft sind, wenn diese Substanzen in Lösung oder unterkühlter Schmelze innerhalb geordneter Strukturen organisiert vorliegen. Dies lässt sich insbesondere dann feststellen, wenn sich ein teilkristalliner Zustand der Glucamide ausbildet. Dieser teilkristalline Zustand ergibt sich aus den speziellen Wechselwirkungen der Glucamide untereinander, welche sich auf die Geometrien der Moleküle selbst und demzufolge natürlich auch auf das Nebenproduktprofil des gewählten Prozessweges zurückführen lassen. Die hier bevorzugt diskutierten teilkristallinen Strukturen zeigen dabei anscheinend eine komplexe Anordnung, welche nicht auf einfache, mizellare Geometriem zurückführbar ist. Teilkristalline Zustände von N-Alkyl-N-Acylglucaminen lassen sich mit den im Stand der Technik genannten Verfahren nicht erhalten und werden insbesondere auch nicht diskutiert.

Es ist insofern die Aufgabe der vorliegenden Erfindung Zusammensetzungen bereitzustellen, welche N-Alkyl-N-Acylglucamine in einer geordneten, teilkristallinen Struktur aufweisen. Weiterhin ist es die Aufgabe der vorliegenden Erfindung ein vorteilhaftes Verfahren zur Herstellung dieser teilkristallinen N-Alkyl-N-Acylglucamin-Zusammensetzungen anzugeben.

Im Sinne dieser Erfindung ist demzufolge eine teilkristalline N-Alkyl-N-Acylglucamin-Zusammensetzung umfassend mindestens zwei unterschiedliche Acylglucamine mit Acylresten ausgewählt aus der Gruppe der gesättigten oder ungesättigten C6-C22 Acyle, dadurch gekennzeichnet, dass die Zusammensetzung bei Raumtemperatur eine teilkristalline Struktur mit mindestens jeweils einen signifikanten Röntgenreflex in den 2Theta-Bereichen ≥ 13,5° bis ≤ 15,1° und ≥ 15,5° bis ≤ 17,4° aufweist, wobei die Röntgenreflexe durch ein Pulver-Diffraktogramm in Bragg-Brentano-Geometrie erhalten werden. Überraschenderweise hat sich herausgestellt, dass auch N-Alkyl-N-Acylglucamine, welche unterschiedliche C-Kettenschnitte aufweisen und zudem in Form von Lösungen oder Mischungen vorliegen, definierte teilkristalline Strukturen ausbilden. Dies im Gegensatz zu N-Alkyl-N-Acylglucamin-Einkristallen, welche aus nur einer definierten N-Alkyl-N-Acylglucamin-Spezies aufgebaut sind und aufgrund der Strukturregelmäßigkeit des einzelnen Glucamid-Bausteins eine regelmäßige Anordnung erwarten lassen. Ohne durch die Theorie gebunden zu sein, können sich diese teilkristallinen, geordneten Strukturen trotz unterschiedlicher Kettenlängen in Lösung oder Mischung auch dann ergeben, wenn das sonstige N-Alkyl-N-Acylglucamin-Nebenproduktspektrum derart vernachlässigbar ist, dass eine genügend hohe Anzahl an Molekülen bereitsteht, um durch Ausbildung geordneter Strukturen die freie Energie des Systems zu erniedrigen. Insbesondere können beispielsweise zyklische (Neben)Produkte eine deutliche Störung von Packungsgeometrien erwarten lassen, welches insgesamt einer teilkristallinen Anordnung entgegenstehen würde. Desweiteren überraschend hat sich herausgestellt, dass diese Struktur im hohen Maße tolerant auf weitere Zusatzstoffe reagiert, ohne den teilkristallinen Zustand zu verlassen. Hierdurch kann sich für die Zusammensetzung eine Reihe von Anwendungsvorteilen ergeben. Oben genannt wurde schon die leichte Verdünnbarkeit durch Zusatz von Lösungsmitteln. Desweiteren kann sich auch ein verbessertes Schmutztragevermögen einstellen, da Schmutzteilchen in größere, teilkristalline Strukturen verlässlicher eingebunden werden können. Dies kann zu einer generell besseren Waschwirkung einer teilkristallinen Zusammensetzung amphiphiler Moleküle beitragen.

Eine teilkristalline Struktur im Erfindungssinne liegt vor, wenn die Zusammensetzung im Rahmen einer Pulver-Diffraktogramm-Aufnahme mindestens zwei definierte Reflexe in den oben angegebenen 2Theta-Winkelbereichen aufweist. Dies im Gegensatz zu nichtteilkristallinen Zusammensetzungen, welche keine geordneten Strukturen in der Zusammensetzung aufweisen, so dass in einer PXRD-Aufnahme nur ein amorpher Halo ohne definierte Peaks gefunden wird. Zur Bestimmung, ob in dem angegebenen Winkelbereich ein signifikanter Reflex vorliegt wird das Kriterium der relativen Intensität herangezogen. Dazu wird die Relation zwischen der Reflexintensität im betrachteten Winkelbereich zur Reflexintensität des stärksten Reflexes im Bereich zwischen 3° und 40° (2Theta) betrachtet. Erst wenn diese Relation oberhalb eines gewissen Schwellenwertes liegt, zum Beispiel 20% (relativ zum stärksten Reflex), liegt im betrachteten Winkelbereich ein signifikanter Reflex vor. Diese Betrachtungsweise berücksichtigt die Verwendung nur arbiträrer Intensitäten (counts/sec). Die Größe der beanspruchten 2Theta-Bereiche ergibt sich aus dem methodenbedingtem Vorliegen von Textureffekten. Die Mindestintensität zur Vorliegen eines signifikanten Reflexes für die angegebenen 2Theta-Bereiche ist für den jeweiligen Strukturtyp in den Beispielen angegeben.

Raumtemperatur im Sinne der Erfindung liegt innerhalb eines Temperaturbereiches zwischen 20 und 25°C.

Die verbesserten Eigenschaften der teilkristallinen Zusammensetzungen lassen sich insbesondere bei Zusammensetzungen mit geordneten Strukturen erzielen, welche signifikante Reflexe in oben angegebenen 2Theta-Bereichen ergeben. Die 2Theta-Bereiche ≥ 13,5° bis ≤ 15,1° und ≥ 15,5° bis ≤ 17,4° deuten auf periodische Strukturen mit mittleren Größenbereichen von 6,1 Å (erster Bereich) und 5,3 Å (zweiter Bereich) hin. Ohne durch die Theorie gebunden zu sein, können diese periodischen Strukturen insbesondere bei Quell- und Umlagerungsvorgängen der kristallinen Strukturen von Bedeutung sein und derart zu verbesserten Wasch- und Handlingseigenschaften beitragen.

Insbesondere können in einer weiteren Ausführungsform der Erfindung die Diffraktogramme der Zusammensetzung einen weiteren signifikanten Reflex aufweisen, welcher einer Zellachsendimension von größer oder gleich 30 Å und kleiner oder gleich 100 Å entspricht. Dieser Reflex zeigt dabei mindestens eine Intensität von größer oder gleich 20 % im Bezug auf die Intensität des stärksten Reflexes im Bereich zwischen 3° und 40° (2Theta).

Eine N-Alkyl-N-Acylglucamin-Zusammensetzung im Sinne der Erfindung kann aus N-Alkyl-N-Acylglucaminen unterschiedlicher Kettenlängen bestehen oder diese enthalten. Zusätzliche Inhaltsstoffe der Zusammensetzungen können beispielsweise weitere Substanzen umfassen, welche im Stand der Technik als in Tensidsystemen einsetzbare, waschaktive oder nichtwaschaktive Substanzen beschrieben werden. Zu dieser Gruppe können allgemein Lösemittel, Builder, Bleichmittel, Enthärter, Enzyme, Duftstoffe, oder Stellmittel gehören. Es hat sich überraschenderweise gezeigt, dass die erfindungsgemäße teilkristalline Zusammensetzung sehr tolerant auf die Aufnahme dieser Inhaltsstoffe reagiert, und sich diese Substanzen weitgehend ohne Verlust der teilkristallinen Struktur integrieren lassen. Insbesondere bevorzugt hingegen ist eine Zusammensetzung, in welcher die waschaktiven Substanzen der Zusammensetzung nur aus N-Alkyl-N-Acylglucaminen bestehen.

Die N-Alkyl-N-Acylglucamine genügen dabei folgender allgemeinen Formel: 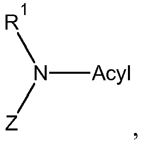 wobei Z für einen Zucker- oder allgemein für einen Polyhydroxyalkylrest, R¹ für einen C1-C4-Alkylrest und Acyl für einen C6-C22 Acylrest der Struktur 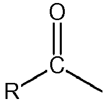 steht, wobei R einen C5-C21 gesättigten oder einfach/mehrfach ungesättigten Alkylrest repräsentiert.

Die Zusammensetzung weist erfindungsgemäß mindestens zwei unterschiedliche Acylglucamine mit Acylresten ausgewählt aus der Gruppe der gesättigten oder ungesättigten C6-C22 Acyle auf. Die Zusammensetzung kann dabei zwischen mindestens 2 und 10, bevorzugt zwischen 2 und 9 und desweiteren bevorzugt zwischen 2 und 8 unterschiedliche N-Alkyl-N-Acylglucamine, welche sich in ihrer Acyl-Kettenlänge unterscheiden, aufweisen. Trotz dieser Anzahl an unterschiedlichen Acyl-Kettenlängen hat sich gezeigt, dass die erfindungsgemäße Zusammensetzung in der Lage ist, eine mindestens teilkristalline Struktur auszubilden, welche periodische Strukturen in den oben angegebenen Bereichen aufweist und demzufolge zu verbesserten Wascheigenschaften beitragen kann. Zur weiteren Vereinfachungen wird in den folgenden Abschnitten die Nomenklatur CX-Glucamide verwendet, wobei beispielsweise die Abkürzung C12-Glucamid für ein N-Alkyl-N-Acylglucamin mit einer Acylkettenlänge von 12 C-Atomen steht.

Die erfindungsgemäßen Zusammensetzungen zeigen dabei zumindest jeweils einen signifikanten Röntgenreflex in den angegebenen 2Theta-Bereichen. Dies bedeutet, dass die Zusammensetzung mindestens eine periodische/teilkristalline Struktur aufweist, welche einer dem Winkelbereich entsprechenden Größenordnung entspricht. Ein signifikanter Röntgenreflex liegt dann vor, wenn die Intensität des Reflexmaximums im Vergleich zur maximalen Intensität des stärksten Signals des Bereiches einen bestimmten Schwellenwert überschreitet. Diese prozentualen Schwellenwerte für die Klassifizierung der einzelnen Röntgenreflexe sind bereichsabhängig und bei den Beispielen angegeben. Die genauen Versuchsbedingungen zur Bestimmung der Reflexlagen in den PXRD-Aufnahmen sind ebenfalls im Rahmen der Beispiele aufgeführt. Die Umrechnung des Winkelbereiches in den Größenbereich ist dabei dem Fachmann bekannt. Desweiteren können in den angegebenen Winkelbereichen aber auch mehr als ein Reflex auftreten. Dies würde dann auf teilkristalline Strukturen mit leicht unterschiedlichen Größenordnungen in diesen Bereich hindeuteten. Auch diese Modifikation soll im Rahmen der vorliegenden Erfindung mit umfasst sein.

Die Bestimmung der Reflexlagen der erfindungsgemäßen Zusammensetzung erfolgt mittels der Aufnahme eines Pulver-Diffraktogramms in Bragg-Brentano-Geometrie. Diese Geometrie ist dem Fachmann zur Aufnahme röntgenographischer Pulverspektren bekannt und hat sich zur verlässlichen Bestimmung der Reflexlagen teilkristalliner Zusammensetzungen als besonders geeignet erwiesen.

In einer zusätzlichen erfindungsgemäßen Ausgestaltung kann die Zusammensetzung bei Raumtemperatur mindestens einen weiteren signifikanten Röntgenreflex in einem 2Theta-Bereich von ≥ 7,5° bis ≤ 8,5° aufweisen. Teilkristalline Zusammensetzungen, welche zusätzlich zu den oben genannten Röntgenreflexen noch mindestens einen weiteren Reflex um circa 8° (2Theta) aufweisen, haben sich im Bereich der Herstellung und in den Anwendungseigenschaften als besonders effizient erwiesen. Ohne durch die Theorie gebunden zu sein, kann sich eine teilkristalline Zusammensetzung mit diesen drei Reflexlagen insbesondere dann ergeben, wenn sehr wenige symmetriestörende Glucamid Nebenprodukte und ein hinreichender Anteil an C12-Acylglucaminen in der Zusammensetzung vorliegen. Diese teilkristalline Struktur hat sich als besonders beständig und flexibel zur Aufnahme weiterer, auch symmetriestörender nicht-Glucamide erwiesen. Zudem kann diese teilkristalline Zusammensetzung unter Beibehalt der Struktur leicht mit weiteren Lösungsmitteln herunter verdünnt werden. Zum Erhalt dieser bevorzugten, teilkristallinen Struktur kann der Anteil der C-12 Acyl-Glucamide an der gesamten Glucamidzusammensetzung desweiteren vorteilhafterweise größer oder gleich 30 mol-% und kleiner oder gleich 85 mol-%, bevorzugt größer oder gleich 35 mol-% und kleiner oder gleich 80 mol-% und weiterhin bevorzugt größer oder gleich 40 mol-% und kleiner oder gleich 75 mol-% betragen.

In einer weiteren Ausgestaltung können teilkristalline Zusammensetzungen besonders geeignet sein, welche neben dem zusätzlichen signifikanten Reflex im Bereich zwischen ≥ 7,5° bis ≤ 8,5°, noch jeweils mindestens einen weiteren signifikanten Reflex in den 2Theta-Bereichen zwischen ≥ 5,6° und ≤ 6,5° und zwischen ≥ 11,6° und ≤ 12,6° aufweisen. Zusammensetzungen mit dieser durch 5 Reflexlagen definierten, teilkristallinen Struktur können sich durch eine sehr gute Waschwirkung und gute Handhabbarkeit auszeichnen.

In einer bevorzugten Ausführungsvariante kann die Zusammensetzung bei Raumtemperatur mindestens einen weiteren signifikanten Röntgenreflex in einem 2Theta-Bereich von ≥ 6,2° bis ≤ 7,5° aufweisen. Eine weitere Untermenge der erfindungsgemäß beanspruchten N-Alkyl-N-Acylglucamine kann dann besonders gute Anwendungseigenschaften zeigen, wenn zusätzlich zu den signifikanten Reflexen bei ≥ 13,5° bis ≤ 15,1° und ≥ 15,5° bis ≤ 17,4° (2Theta) mindestens ein weiterer signifikanter Reflex zwischen ≥ 6,2° und ≤ 7,5° auffindbar ist. Ohne durch die Theorie gebunden zu sein können sich diese vorteilhaften Zusammensetzungen in den Fällen ergeben, in welchen der Anteil der C12-Glucamide im Rahmen der Zusammensetzungen gering ist. Diese Zusammensetzungen können insbesondere sehr stabile Schaumeigenschaften zeigen und können vorteilhafterweise größer oder gleich 0 mol-% und kleiner oder gleich 3 mol-%, bevorzugt größer oder gleich 0 mol-% und kleiner oder gleich 2 mol-% und weiterhin bevorzugt größer oder gleich 0 mol-% und kleiner oder gleich 1 mol-% C12-Glucamide beinhalten.

In einer alternativen Ausführungsvariante kann die teilkristalline Zusammensetzung C12 N-Methyl-N-Acylglucamin umfassen und der C-12 Acylglucamin-Anteil bezogen auf den gesamten N-Methyl-N-Acylglucamin-Gehalt größer oder gleich 25 mol-% und kleiner oder gleich 95 mol-% betragen. Innerhalb dieser Ausführungsform besteht die Zusammensetzung aus mehreren N-Alkyl-N-Acylglucaminen, wobei der Anteil des C12-Methyl-Glucamid-Kettenschnitts im oben angegebenen Bereich liegt. Zusammensetzungen mit einem C12-Acyl-Methyl-Glucamin-Gehalt in diesen Mengenbereichen haben sich dabei als besonders balanciert im Rahmen der Waschwirkung, Löslichkeit und des Schaumvermögens herausgestellt. Dies hochwahrscheinlich bedingt durch eine besonders günstige, mittlere Kettenlänge des gesamten Kettenschnitts der Zusammensetzung. Zum Erhalt dieser bevorzugten, teilkristallinen Struktur kann der Anteil der C-12 Acyl-Methyl-Glucamide an der Gesamtzusammensetzung desweiteren vorteilhafterweise größer oder gleich 30 mol-% und kleiner oder gleich 90 mol-%, bevorzugt größer oder gleich 35 mol-% und kleiner oder gleich 85 mol-% und weiterhin bevorzugt größer oder gleich 40 mol-% und kleiner oder gleich 80 mol-% betragen.

Eine weitere bevorzugte Ausführungsform bezieht sich auf eine Zusammensetzung, welche zusätzlich ein Lösemittel ausgewählt aus der Gruppe der ein-, zwei- oder dreiwertigen Alkohole umfasst. Diese Gruppe an Lösemitteln hat sich als besonders günstig zur Verdünnung der erfindungsgemäßen Zusammensetzungen erwiesen, wobei diese Lösemittel in weiten Konzentrationsbereichen zugesetzt werden können, ohne die teilkristalline Struktur der N-Alkyl-N-Acylglucamine zu verlieren. Ohne durch die Theorie gebunden zu sein können sich diese Mono- oder Polyole symmetriegünstig zwischen die hochwahrscheinlich assoziierten, hydrophilen Zuckerreste der N-Alkyl-N-Acylglucamine einlagern, sodass es ggf. nur zu einer geringfügigen Aufweitung der beobachtbaren kristallinen Strukturen kommen kann. Es können beispielsweise größer oder gleich 2,5 Gew-% und kleiner oder gleich 30 Gew-%, bevorzugt größer oder gleich 5 Gew-% und kleiner oder gleich 25 Gew-% und desweiteren bevorzugt größer oder gleich 10 Gew-% und kleiner oder gleich 20 Gew-% aus dieser Gruppe an Lösemitteln eingelagert werden. Im Rahmen einer besonderen Ausführungsform kann das Lösemitte entweder aus Glycerin oder Propylenglycol oder Mischungen daraus bestehen oder diese enthalten. Dies hat sich als besonders günstig in Bezug auf die Fließfähigkeit der Zusammensetzung erwiesen.

In einer weiteren Ausgestaltung der Erfindung kann die Konzentration der N-Alkyl-N-Acylglucamine in der Zusammensetzung größer oder gleich 5 Gewichts-% und kleiner oder gleich 65 Gewichts-% betragen. Dieser Anteil an N-Alkyl-N-Acylglucaminen hat sich zur Ausbildung zumindest teilkristalliner Strukturen als besonders vorteilhaft erwiesen. Die Wechselwirkungen der erfindungsgemäßen Zusammensetzungen sind dabei anscheinend derart ausreichend, dass auch größere Mengen an nicht N-Alkyl-N-Acylglucaminen zu keiner großen Störung der geordneten Strukturen führen. Insbesondere kann die Konzentration der N-Alkyl-N-Acylglucamine in der Zusammensetzung desweiteren größer oder gleich 10 Gewichts-% und kleiner oder gleich 60 Gewichts-%, bevorzugt größer oder gleich 15 Gewichts-% und kleiner oder gleich 50 Gewichts-% und weiterhin bevorzugt größer oder gleich 25 Gewichts-% und kleiner oder gleich 55 Gewichts-% betragen. Diese Konzentrationsbereiche ermöglichen unter anderem eine effektive Logistik in der Herstellungskette. Im Rahmen kleiner N-Alkyl-N-Acylglucamin-Konzentrationen kann es sich ggf. auch anbieten, dass die PXRD-Untersuchungen mittels Kapillaren durchgeführt werden. Dies kann bei geringen Signalstärken die Statistik der Messungen erhöhen.

In einer weiteren Charakteristik kann der Anteil mindestens eines N-Alkyl-N-Acylglucamins in der Zusammensetzung bezogen auf den gesamten N-Alkyl-N-Acylglucamin-Gehalt größer oder gleich 40 mol-% und kleiner oder gleich 95 mol-% betragen. Überraschenderweise hat sich gezeigt, dass die Zusammensetzung zur Ausbildung einer mindestens teilkristallinen Struktur nicht zwangsläufig aus nur einer N-Alkyl-N-Acylglucamin-Spezies bestehen muss. Dies ist überraschend, da sich regelmäßige Strukturen dann leichter ausbilden, wenn die Symmetrie der Einzelbausteine gleich ist. Störungen der Symmetrie, z.B. durch unterschiedliche Acyl-Kettenlängen sollten zu einem unterschiedlichen Raumbedarf der Ketten führen, welches erst einmal einer teilkristallinen Anordnung entgegenstehen sollte. Eine besonders günstige Anlagerung der N-Alkyl-N-Acylglucamine, und daraus folgend auch besonders günstige Anwendungseigenschaften, haben sich in oben angegebener relativer Zusammensetzung ergeben. Weitere bevorzugte Ausgestaltungen können sich auch dann ergeben, wenn der Anteil mindestens eines N-Alkyl-N-Acylglucamins in der Zusammensetzung bezogen auf den gesamten N-Alkyl-N-Acylglucamin-Gehalt größer oder gleich 45 mol-% und kleiner oder gleich 85 mol-%, bevorzugt größer oder gleich 45 mol-% und kleiner oder gleich 75 mol-% beträgt. Diese Relationen können eine Zusammensetzung mit zumindest teilkristalliner Struktur ergeben und erlauben eine Adaption der einzelnen Produkte auf die jeweils gewünschten Anwendungseigenschaften.

Des Weiteren erfindungsgemäß ist ein Verfahren zur Herstellung einer teilkristallinen N-Alkyl-N-Acylglucamin-Zusammensetzung umfassend die Schritte:
a) Zur Reaktion bringen des N-Alkyl-Glucamins mit einer Base in wässriger Lösung,
b) Trocknen des Reaktionsproduktes aus Schritt a),
c) Vorlegen mindestens zweier Carbonsäurealkylester mit unterschiedlicher C6-C22-Kettenlänge,
d) Portionsweises Hinzufügen des unter b) erhaltenen, getrockneten Reaktionsproduktes zu der Mischung aus c) und
e) Ausreagieren der Zusammensetzung aus d) unter reduziertem Druck.
Diese Herstellungsart hat sich als besonders effizient und nebenproduktarm herausgestellt, sodass sich durch diesen Prozess N-Alkyl-N-Acylglucamine darstellen lassen, welche besonders zur Ausbildung mindestens teilkristalliner Strukturen geeignet sind. Ohne durch die Theorie gebunden zu sein, kann durch die Kombination der Schritte a) und b) ein basisches N-Alkyl-Glucamin erhalten werden, welches hochwahrscheinlich das Zentrum der basischen Eigenschaften am Stickstoff aufweist. Dieser basische Stickstoff kann im Besonderen dazu geeignet sein, hoch selektiv mit den Carbonsäurealkylestern unter basischer Spaltung derselben zu reagieren. Durch diese Verfahrensführung kann anscheinend die Ausbildung unerwünschter, sterisch unterschiedlicher, beispielsweise zyklischer, Nebenprodukte derart zurückgedrängt werden, so dass sich sterisch sehr ähnlich Produkte mit erhöhter Fähigkeit zur Ausbildung teilkristalliner Strukturen ergeben. Dieses kann für den Erhalt verbesserter Anwendungseigenschaften vorteilhaft sein. Der Anteil an unerwünschten Nebenprodukten und nicht reagierten Ausgangsmaterial wird auch von anderen Patentdokumenten, zum Beispiel der WO9410130 A1, als Nachteil der bekannten Verfahren benannt.

Zur Synthese der N-Alkyl-N-Acylglucamine können als N-Alkyl-Glucamine insbesondere N-Alkyl-Glucamine mit einer Alkylgruppe R¹ ausgesucht aus der Gruppe der C1-C4-Alkyle verwendet werden. Diese Alkyl-Reste umfassen insbesondere Methyl-, Ethyl-, n-Propyl-, isoPropyl-, n-Butyl-, iso-Butyl-, sec.-Butyl-, tert.-Butyl-Gruppen. Zum Erhalt zumindest teilkristalliner Zusammensetzungen können dabei bevorzugt Methyl, Ethyl, n-Propyl und n-Butyl eingesetzt werden. Diese N-Alkylgruppen können zu besonders packungsfreundlichen N-Alkyl-N-Acylglucaminen führen. Besonders anwendungsfreundliche N-Alkyl-N-Acylglucamine können sich zudem durch die Verwendung von n-Methyl-Glucaminen ergeben. Aus diesem Grund kann der Einsatz von N-Methyl-Glucamin bevorzugt sein.

Der Polyhydroxyalkylrest Z der N-Alkyl-Glucamine kann sich von Monosacchariden wie Erythrose, Threose, Ribose, Arabinose, Xylose, Lyxose, Allose, Altrose, Glucose, Mannose, Gulose, Idose, Galactose, Talose oder Fructose oder Derivaten hiervon wie Glucuronsäure oder Desoxyribose oder von Oligosacchariden, insbesondere von Disacchariden wie Saccharose, Lactose, Trehalose, Maltose, Cellobiose oder Gentiobiose, daneben auch von Trisacchariden wie Raffinose, ableiten. Weiterhin kommen alle technischen Stärkeabbauprodukte wie Glucosesirup oder Dextrine, z.B. Maltodextrine, als Ausgangmaterial für die Gruppe Z in Betracht. Bevorzugt ergibt sich für die Variable Z ein von Aldohexosen abgeleiteter Polyhydroxyalkylrest der Formel -CH₂-(CHOH)₄-CH₂OH. Besonders bevorzugt kann dies ein Rest der Glucose, insbesondere der D-Glucose, sein.

Als Base in wässriger Lösung können im erfindungsgemäßen Verfahren Alkoxide und/oder Alkalimetallhydoxide eingesetzt werden. Bevorzugt können insbesondere C1-C4 Alkoxide mit Alkalimetall-Gegenionen oder Alkalimetall-Hydroxide wie beispielsweise LiOH, NaOH KOH oder deren Mischungen eingesetzt werden. Im Rahmen des Verfahrens kann die Konzentration der Basen in der wässrigen Lösung größer oder gleich 0,05 N und kleiner oder gleich 5 N, bevorzugt größer oder gleich 0,05 N und kleiner oder gleich 4 N und desweiteren bevorzugt größer oder gleich 0,05 N und kleiner oder gleich 3 N betragen. Innerhalb dieses Konzentrationsbereiches lassen sich hohe Umsetzungsgeschwindigkeiten des N-Alkyl-Glucamins erhalten.

Im Schritt c) werden mindestens zwei Carbonsäurealkylester mit unterschiedlicher C6-C22-Kettenlänge eingesetzt. Die Carbonsäurealkylester genügen der allgemeinen Formel R-CO-O-R², wobei R² einen C1 bis C4 Alkylrest wie beispielsweise Methyl-, Ethyl-, n-Propyl-, isoPropyl-, n-Butyl-, iso-Butyl-, sec.-Butyl-, tert.-Butyl repräsentiert. Bevorzugt können als Akylreste der Carbonsäurealkylester Methyl- und Ethylester eingesetzt werden. Als Carbonsäurealkylester lassen sich erfindungsgemäß auch Mono-, Di- oder Triglyceride mit unterschiedlichen C6-C22-Kettenlängen einsetzen. Insbesondere lassen sich auch aus Triglyceridölen wie beispielsweise Kokos- oder Palmkernöl durch das erfindungsgemäße Verfahren teilkristalline Glucamid-Zusammensetzungen erhalten.

Der Alkylrest R des Carbonsäuresegmentes kann einen C5-C21 gesättigten oder einfach/mehrfach ungesättigten Alkylrest repräsentieren. Bevorzugt kann der Carbonsäureester aus einer langkettigen, natürlich vorkommenden Fettsäure wie beispielsweise Laurin-, Myristin-, Palmitin- und/oder Stearinsäure durch Veresterung gebildet worden sein. Desweiteren kann insbesondere dann eine Mischung mindestens zweier Carbonsäurealkylester vorliegen, wenn der Anteil jedes Carbonsäurealkylesters mindestens größer oder gleich 5 mol-%, bevorzugt größer oder gleich 10 mol-% und desweiteren bevorzugt größer oder gleich 15 mol-% an der Carbonsäurealkylester-Gesamtmenge ausmacht. Trotz dieser Mischung unterschiedlicher C-Kettenlängen können sich teilkristalline Glucamid-Strukturen ausbilden.

Des Weiteren kann in einer zusätzlichen Ausgestaltung des Verfahrens die Base aus Schritte a) Natronlauge sein. Natronlauge hat sich im Rahmen der Prozessführung zur effizienten Konditionierung des N-Alkyl-Glucamins als besonders geeignet herausgestellt. Im Rahmen des Verfahrens kann die NaOH-Konzentration in der Umsetzungslösung größer oder gleich 0,05 N und kleiner oder gleich 5 N, bevorzugt größer oder gleich 0,1 N und kleiner oder gleich 3 N und desweiteren bevorzugt größer oder gleich 0,2 N und kleiner oder gleich 2 N betragen. Dies kann zu sehr selektiven Umsetzungen mit nur einem sehr geringen Anteil an unreagiertem N-Alkyl-Glucamin führen.

Gemäß einer weiteren Ausgestaltung des erfindungsgemäßen Verfahrens kann der Wassergehalt des getrockneten Reaktionsproduktes nach Schritt b) größer oder gleich 0,05 Gewichts-% und kleiner oder gleich 0,4 Gewichts-% betragen. Überraschenderweise hat sich gezeigt, dass eine komplette Trocknung des Reaktionsproduktes aus Schritt a) für die weitere Verfahrensführung nachteilig sein kann. Geringe Anteile an Wasser nach der Trocknung scheinen in der Lage zu sein, die Reaktion zu beschleunigen. Desweiteren scheint der Restwassergehalt auch Auswirkungen auf die Regioselektivität der anschließenden Reaktion zu besitzen. Aus diesem Grund kann desweiteren bevorzugt ein Restwassergehalt des Reaktionsproduktes nach Schritt b) von größer oder gleich 0,1 Gewichts-% und kleiner oder gleich 0,4 Gewichts-% und desweiteren bevorzugt von größer oder gleich 0,15 Gewichts-% und kleiner oder gleich 0,35 Gewichts-% vorliegen. Größere Wassermengen können sich dagegen nachteilig auf die Ausbildung der teilkristallinen Struktur auswirken. Der Restwassergehalt in dieser Stufe kann nach dem Fachmann bekannten Verfahren erfolgen. Zweckmäßigerweise kann der Nachweis durch eine Karl-Fischer-Titration des getrockneten Produktes erfolgen.

In einer weiteren Charakteristik des erfindungsgemäßen Verfahrens können die Carbonsäurealkylester in Schritt c) in einem Lösemittel ausgewählt aus der Gruppe umfassend ein-, zwei- und/oder dreiwertige Alkohole vorgelegt werden. Das Ausführen der Reaktion in Schritt c) in Gegenwart eines ein- oder mehrwertigen Alkohols kann zu einer Verringerung der Viskosität der Reaktionslösung und damit zu einer schnelleren und vollständigeren Reaktion beitragen. Gegebenenfalls lässt sich auch durch den Einsatz der Alkohole eine gleichmäßigere Temperaturführung erreichen. Dies kann Einfluss auf die Vollständigkeit der Eduktumsetzung, sowie auf das Nebenproduktprofil haben. Bevorzugt kann der Schritt c) in Diolen erfolgen, wobei Propylenglykol bevorzugt eingesetzt werden kann. Adäquate Konzentrationsbereiche für die Lösungsmittel können sich in Bereichen zwischen größer oder gleich 5 Gew-% und kleiner oder gleich 70 Gew-%, vorzugsweise zwischen größer oder gleich 10 Gew-% und kleiner oder gleich 50 Gew-% und weiterhin bevorzugt zwischen größer oder gleich 10 Gew-% und kleiner oder gleich 40 Gew-% ergeben.

Zusätzlich kann in einem weiteren Aspekt des erfindungsgemäßen Verfahrens der Verfahrensschritt e) in einem Temperaturbereich von größer oder gleich 50 °C und kleiner oder gleich 175 °C durchgeführt werden. Das Ausreagieren der Zusammensetzung kann vorzugsweise unter Hitzeeinwirkung erfolgen. Eine effiziente Verfahrensführung hat sich dabei in dem oben angegebenen Temperaturbereich ergeben. Ebenfalls gute Resultate lassen sich aber auch dadurch erhalten, dass die Mischung in einem Temperaturbereich von größer oder gleich 50 °C und kleiner oder gleich 150 °C, bevorzugt von größer oder gleich 70 °C und kleiner oder gleich 130 °C und desweiteren bevorzugt von größer oder gleich 85 °C und kleiner oder gleich 110 °C ausreagiert. Innerhalb dieses Temperaturbereiches kann sich eine effiziente und vollständige Umsetzung mit sehr geringen Nebenprodukt-Konzentrationen ergeben.

Zweckmäßigerweise wird der Verfahrensschritt e) unter reduziertem Druck durchgeführt. Mittels des reduzierten Druckes können dabei flüchtige Reaktionsbestandteile abgezogen werden. Zum Erhalt eines möglichst kleinen Anteils an Beiprodukten kann der Druck auf größer oder gleich 10 mbar und kleiner oder gleich 100 mbar und desweiteren bevorzugt auf größer oder gleich 25 mbar und kleiner oder gleich 50 mbar eingeregelt werden. Damit lassen sich zum Beispiel niedrig siedende Alkohole aus der Reaktionsmischung entfernen und sich so das Reaktionsgleichgewicht in Richtung der Produkte verschieben.

In einer weiteren Ausgestaltung des erfindungsgemäßen Verfahrens kann das Reaktionsprodukt aus Schritt e) in einem zusätzlichen Verfahrensschritt durch Zugabe mindestens eines protischen Lösemittels ausgewählt aus der Gruppe umfassend Wasser, ein-, zwei-, und/oder dreiwertige Alkohole oder Mischungen daraus, auf ein N-Alkyl-N-Acylglucamin-Gehalt von größer oder gleich 2,5 Gewichts-% und kleiner oder gleich 50 Gewichts-% eingestellt werden. Überraschenderweise lassen sich die erfindungsgemäßen Zusammensetzungen unter Beibehalt ihrer zumindest teilkristallinen Struktur verdünnen. Dies kann zweckmäßigerweise durch die Zugabe der oben genannten Lösemittel erfolgen. Ohne durch die Theorie gebunden zu sein ist dies hochwahrscheinlich deshalb möglich, da der Anteil an unerwünschten Nebenprodukten durch die gewählte Verfahrensführung äußerst gering ist. Aus diesem Grund können die Wechselwirkungen zwischen den N-Alkyl-N-Acylglucaminen besonders hoch sein, sodass auch eine An-/Einlagerung weiterer Lösemittelmoleküle erfolgen kann, ohne die teilkristallinen Strukturen zu zerstören.

Erfindungsgemäß ist weiterhin die Verwendung einer teilkristallinen N-Alkyl-N-Acylglucamin-Zusammensetzung in Reinigungs-, kosmetischen, dermatologischen und/oder pharmazeutischen Anwendungen. Es hat sich vorteilhafterweise herausgestellt, dass sich die teilkristallinen Zusammensetzungen aufgrund ihres Eigenschaftsprofiles besonders für die Verwendung in oben genannten Bereichen eigenen. Die Zusammensetzungen sind dermatologisch hoch akzeptabel, zeigen gute Schaum- und Reinigungseigenschaften und sind biologisch abbaubar. Ohne durch die Theorie gebunden zu sein kann sich die hohe dermatologische Akzeptanz auch durch das Ausbilden der teilkristallinen Strukturen ergeben. Möglicherweise kann die teilkristalline Anordnung der Glucamide die Diffusion in und die Interaktion mit tieferen Hautstrukturen derart verringern, sodass die Barrierewirkung der Haut in einem hohen Maße erhalten bleibt. Dies im Gegensatz zu Tensid-Mizellen oder einzelnen Tensidmolekülen anderer Amphiphile, welche ungehindert, zum Beispiel durch Hautporen oder die Lipidschichten, in tiefere Dermisschichten penetrieren können.

Insbesondere kann innerhalb eines weiteren Verwendungsaspektes die Reinigungsanwendung eine Rinse-off Personal Care-Anwendung sein. Insbesondere können sich die erfindungsgemäßen Zusammensetzungen aufgrund ihrer Viskositäts- und Schaumeigenschaften als Reinigungsmittel anbieten. Dies insbesondere bedingt durch ihre dermatologische Kompatibilität mit Hautoberflächen oder auch den Haaren. Werden zudem hocheffektive Reinigungssysteme nachgefragt, welche innerhalb einer sehr kurzen Zeitspanne effektiv arbeiten sollen, können sich diese Zusammensetzungen gerade in Bereich von nach der Applikation abwaschbaren "rinse-off" Produkten anbieten.

Hinsichtlich weiterer Vorteile und Merkmale der vorbeschriebenen Verwendung wird hiermit explizit auf die Erläuterungen im Zusammenhang mit der erfindungsgemäßen Zusammensetzung sowie dem erfindungsgemäßen Verfahren verwiesen. Auch sollen erfindungsgemäße Merkmale und Vorteile des erfindungsgemäßen Verfahrens auch für die erfindungsgemäße Zusammensetzung und die erfindungsgemäße Verwendung anwendbar sein und als offenbart gelten und umgekehrt. Unter die Erfindung fallen auch sämtliche Kombinationen aus zumindest zwei von in der Beschreibung und/oder den Ansprüchen offenbarten Merkmalen.

Die Erfindung wird nachfolgend anhand von Ausführungsbeispielen näher erläutert.

Für die Pulverröntgenbeugung (PXRD) wurden die Proben auf einem rotierenden ,zerobackground' Probenträger aus Silizium präpariert. Die Messungen erfolgten in Reflektion bei Raumtemperatur (20 - 25 °C) mit einem Bruker D8 Advance-Diffraktometer (Cu-Kα1 = 1,54059 Å; Johansson Primärstrahl Monochromator, positionsempfindlichen Detektor). Die Messzeit betrug jeweils 2 Stunden.
Zur Auswertung der Daten wurde das Programm EVA Version 14.0 der Firma Bruker-AXS verwendet. Die Ermittlung der Peakintensitäten in counts/sec wurde eine Untergrundkorrektur mit den Programmeinstellungen Threshold=1 sowie Curvature=1 vorgenommen. Eine Normierung fand mit jeweils dem Signalmaximum (100%) der höchsten Intensität (counts/sec) im 2Theta Bereich 3-40° statt.

Signifikante Reflexe liegen im Erfindungssinne dann vor, wenn die Relation zwischen maximal beobachteter Intensität im betrachteten Winkelbereich größer oder gleich ist, wie der angegebene Anteil an der höchsten gemessenen Intensität im 2Theta-Bereich zwischen 3° und 40°. Es ergeben sich folgende Relationen:

| Betrachteter 2Theta-Bereich | %-Anteil der Intensität in Relation zum Intensitätsmaximum (3°-40°) |
|---|---|
| ≥ 15,5° bis ≤ 17,4° | ≥ 10% |
| ≥ 13,5° bis ≤ 15,1° | ≥ 5% |
| ≥ 11,6 bis ≤ 12,6 | ≥ 7% |
| ≥ 7,5° bis ≤ 8,5° | ≥ 20 % |
| ≥ 6,2° bis ≤ 7,5° | ≥ 5 % |
| ≥ 5,6° bis ≤ 6,5° | ≥ 3 % |

An zahlreichen unterschiedlichen N-Methyl-Glucamiden, welche mittels des erfindungsgemäßen Verfahrens erhalten wurden, wurden mittels oben angegebener Methode PXRD-Spektren aufgenommen.

Es zeigen:
Fig. 1 das PXRD-Spektrum einer erfindungsgemäßen Zusammensetzung mit einem C8/C10-Glucamid Kettenschnitt
Fig. 2 das PXRD-Spektrum einer erfindungsgemäßen Zusammensetzung mit einem C12/C14-Glucamid Kettenschnitt
Fig. 3 das PXRD-Spektrum einer erfindungsgemäßen Zusammensetzung mit einem C 16/C 18-Glucamid Kettenschnitt
Fig. 4 das PXRD-Spektrum einer erfindungsgemäßen Zusammensetzung mit einem C12/C14/C16/C18'-Glucamid Kettenschnitt
Fig. 5 das PXRD-Spektrum einer erfindungsgemäßen Zusammensetzung mit einem C8/C 10/C12/C14/C 16/C18/C18'-Glucamid Kettenschnitt

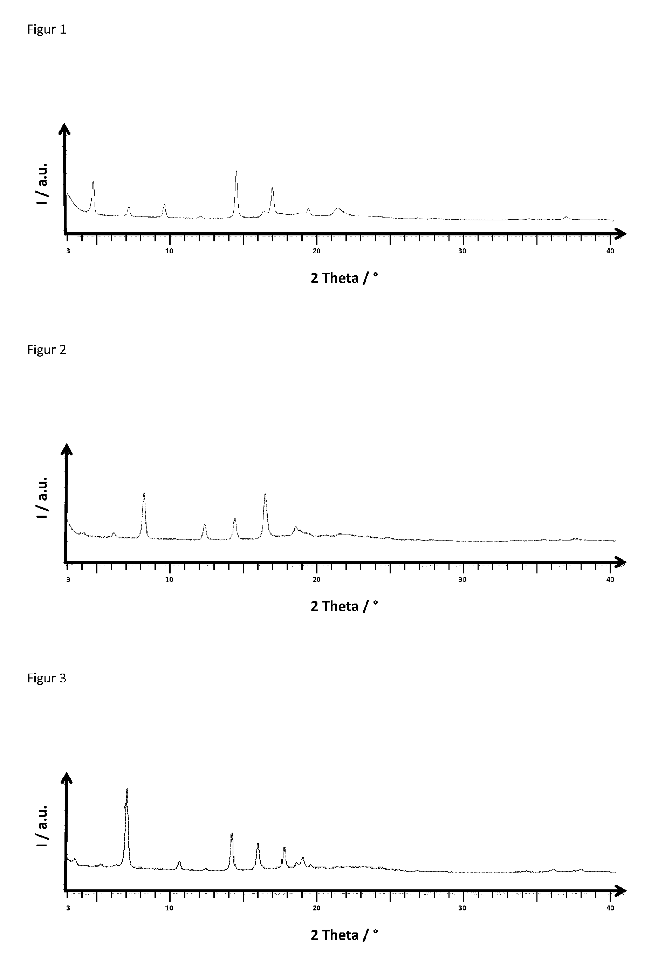

Figur 1 zeigt ein in Bragg-Brentano-Geometrie aufgenommenes Röntgen-Pulverdiffraktogramm einer erfindungsgemäßen teilkristallinen N-Alkyl-N-Acylglucamin-Zusammensetzung im Bereich zwischen 3° und 40° (2Theta). Deutlich lassen sich die teilkristallinen Strukturen anhand der Reflexe in den 2Theta-Bereichen ≥ 13,5° bis ≤ 15,1° und ≥ 15,5° bis ≤ 17,4° erkennen. Die Probe stellt einen C8/C10 N-Methyl-Glucamid-Kettenschnitt dar, wobei 40 mol-% der Glucamide einen C8- und 60 mol-% der Glucamide einen C10-Kettenrest tragen. Der Anteil an Glucamiden an der Probe beträgt ca. 90 Gewichts-%. Der Rest der Probe (ca. 10 Gewichts-%) umfasst Propylenglykol. Gegebenenfalls können auch Spuren von Wasser enthalten sein.

Figur 2 zeigt ein in Bragg-Brentano-Geometrie aufgenommenes Röntgen-Pulverdiffraktogramm einer erfindungsgemäßen teilkristallinen N-Alkyl-N-Acylglucamin-Zusammensetzung im Bereich zwischen 3° und 40° (2Theta). Deutlich lassen sich die teilkristallinen Strukturen anhand der Reflexe in den 2Theta-Bereichen ≥ 13,5° bis ≤ 15,1° und > 15,5° bis ≤ 17,4° erkennen. Die Probe stellt einen C12/C14 N-Methyl-Glucamid-Kettenschnitt dar, wobei 75 mol-% der Glucamide einen C12- und 25 mol-% der Glucamide einen C14-Kettenrest tragen. Zusätzlich zeigt das Diffraktogramm noch einen signifikanten Röntgenreflex im 2Theta-Bereich von ≥ 7,5° bis ≤ 8,5°. Der Anteil an Glucamiden an der Probe beträgt ca. 90 Gewichts-%. Der Rest der Probe (ca. 10 Gewichts-%) umfasst Propylenglykol. Gegebenenfalls können auch Spuren von Wasser enthalten sein.

Figur 3 zeigt ein in Bragg-Brentano-Geometrie aufgenommenes Röntgen-Pulverdiffraktogramm einer erfindungsgemäßen teilkristallinen N-Alkyl-N-Acylglucamin-Zusammensetzung im Bereich zwischen 3° und 40° (2Theta). Deutlich lassen sich die teilkristallinen Strukturen anhand der Reflexe in den 2Theta-Bereichen ≥ 13,5° bis ≤ 15,1° und ≥ 15,5° bis ≤ 17,4° erkennen. Zusätzlich zeigt das Diffraktogramm noch einen signifikanten Röntgenreflex in einem 2Theta-Bereich von ≥ 6,2° bis ≤ 7,5°. Die Probe stellt einen C16/C18 N-Methyl-Glucamid-Kettenschnitt dar, wobei 60 mol-% der Glucamide einen C16- und 40 mol-% der Glucamide einen C18-Kettenrest tragen. Der Anteil an Glucamiden an der Probe beträgt ca. 80 Gewichts-%. Der Rest der Probe (ca. 20 Gewichts-%) umfasst Propylenglykol. Gegebenenfalls können auch Spuren von Wasser enthalten sein.

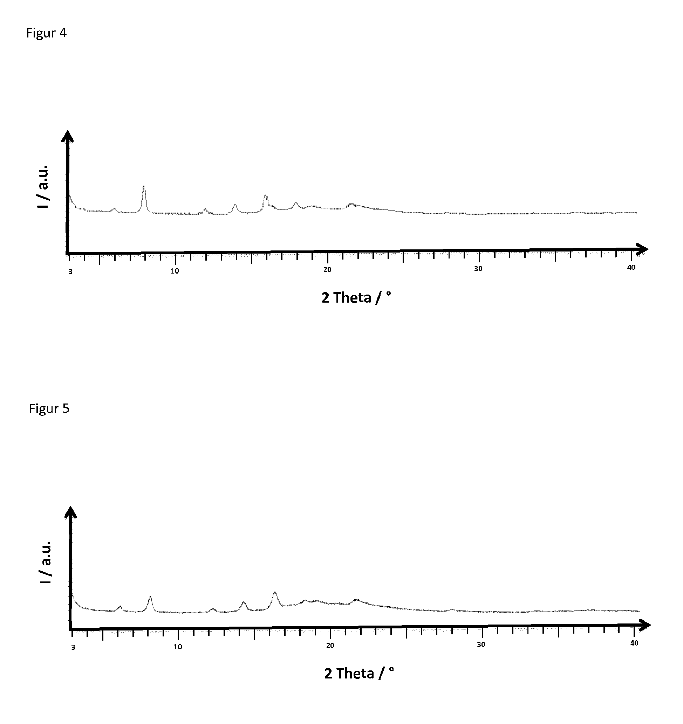

Figur 4 zeigt ein in Bragg-Brentano-Geometrie aufgenommenes Röntgen-Pulverdiffraktogramm einer erfindungsgemäßen teilkristallinen N-Alkyl-N-Acylglucamin-Zusammensetzung im Bereich zwischen 3° und 40° (2Theta). Deutlich lassen sich die teilkristallinen Strukturen anhand der Reflexe in den 2Theta-Bereichen ≥ 13,5° bis ≤ 15,1° und > 15,5° bis ≤ 17,4° erkennen. Die Probe stellt einen C12/C14/C16/C18/C18' N-Methyl-Glucamid-Kettenschnitt dar, wobei 62 mol-% der Glucamide einen C12-, 24 mol-% der Glucamide einen C14-, 5 mol-% der Glucamide einen C16-, 1 mol-% der Glucamide einen C18- und 8 mol-% der Glucamide einen C18'-Kettenrest tragen. Die Bezeichnung C18'-Kettenrest besagt, dass der C18'-Kettenrest mindestens eine ungesättigte Doppelbindung trägt. Es findet sich überraschenderweise trotz der Doppelbindung in einigen der Ketten eine teilkristalline Struktur. Dies ist insbesondere überraschend, da Doppelbindungen durch ihre eingeschränkte Rotation normalerweise eine Ausbildung kristalliner Strukturen deutlich erschweren können. Der Anteil an Glucamiden an der Probe beträgt ca. 88 Gewichts-%. Der Rest der Probe (ca. 12 Gewichts-%) umfasst Propylenglykol. Gegebenenfalls können auch Spuren von Wasser enthalten sein.

Figur 5 zeigt ein in Bragg-Brentano-Geometrie aufgenommenes Röntgen-Pulverdiffraktogramm einer erfindungsgemäßen teilkristallinen N-Alkyl-N-Acylglucamin-Zusammensetzung im Bereich zwischen 3° und 40° (2Theta). Deutlich lassen sich die teilkristallinen Strukturen anhand der Reflexe in den 2Theta-Bereichen ≥ 13,5° bis ≤ 15,1° und ≥ 15,5° bis ≤ 17,4° erkennen. Die Probe stellt einen C8/C10/C12/C14/C16/C18/C18' N-Methyl-Glucamid-Kettenschnitt dar, wobei 8 mol-% der Glucamide einen C8-, 6 mol-% der Glucamide einen C10-, 48 mol-% der Glucamide einen C12-,18 mol-% der Glucamide einen C14-, 9 mol-% der Glucamide einen C16-, 3 mol-% der Glucamide einen C 18- und 8 mol-% der Glucamide einen C18'-Kettenrest tragen. Die Bezeichnung C18'-Kettenrest besagt, dass der C18'-Kettenrest mindestens eine ungesättigte Doppelbindung trägt. Der Anteil an Glucamiden an der Probe beträgt ca. 83 Gewichts-%. Der Rest der Probe (ca. 17 Gewichts-%) umfasst Propylenglykol und Glycerin. Gegebenenfalls können auch Spuren von Wasser enthalten sein.

### Darstellung der N-Alkyl-N-Acylglucamine:

In einem ersten Schritt wird ein N-Alkyl-Glucamin, beispielsweise N-Methyl-Glucamin (NMG), mit konzentrierter, wässriger Natronlauge zusammengegeben. Die Konzentration des Natriumhydroxids in der Zusammensetzung kann dabei beispielsweise zwischen 0,5 und 1,5 Gewichts-% liegen. Man lässt die Vorstufe für mindestens 2 h ausreagieren und trocknet die Vorstufe anschließend unter Wärmezufuhr. Gegebenenfalls kann die Trocknung auch durch Anlegen eines partiellen Vakuums gefördert werden. Bevorzugt wird die Vorstufe dabei nicht vollständig entwässert.

In einem zweiten Verfahrensschritt werden mindestens zwei Carbonsäurealkylester mit unterschiedlicher C-Kettenlänge oder Mono-, Di- oder Triglyceride mit unterschiedlichen C-Kettenlängen vorgelegt. Das Vorlegen der Carbonsäurealkylester kann auch in einem Lösungsmittel, wie beispielsweise Propylenglykol, erfolgen. Die Carbonsäurealkylester oder das Carbonsäurealkylester/Lösungsmittel werden auf eine Temperatur zwischen 50 °C und kleiner oder gleich 150 °C erhitzt. Beispielsweise kann die Zusammensetzung auf 100 °C erwärmt werden. Zu diesem erwärmten Gemisch wird nun die getrocknete Vorstufe aus dem Schritt 1) portionsweise hinzugefügt. Gegebenenfalls können leicht flüchtige Reaktionsprodukte durch ein partielles Vakuum, beispielsweise in einem Druckbereich zwischen 25 mbar - 50 mbar, aus der Reaktionsmischung entfernt werden. Nach Zugabe der gesamten Vorstufe lässt die Mischung für weitere 2 Stunden ausreagieren und man erhält die N-Alkyl-Glucamide. Je nach Verfahrensführung kann die N-Alkyl-Glucamid-Zusammensetzung ein Lösungsmittel, beispielsweise Propylenglykol, und bei Einsatz von Triglyceriden, ggf. Glycerin enthalten.

In einem weiteren Verfahrensschritt kann die Zusammensetzung durch Wasser oder weitere organische Lösungsmittel wie Alkohole unter Eintrag von Rührenergie weiter verdünnt werden.

## Patentansprüche

1. Teilkristalline N-Alkyl-N-Acylglucamin-Zusammensetzung umfassend mindestens zwei unterschiedliche Acylglucamine mit Acylresten ausgewählt aus der Gruppe der gesättigten oder ungesättigten C6-C22 Acyle, **dadurch gekennzeichnet, dass** die Zusammensetzung bei Raumtemperatur eine teilkristalline Struktur mit mindestens jeweils einen signifikanten Röntgenreflex in den 2Theta-Bereichen ≥ 13,5° bis ≤ 15,1° und ≥ 15,5° bis ≤ 17,4° aufweist, wobei die Röntgenreflexe durch ein Pulver-Diffraktogramm in Bragg-Brentano-Geometrie erhalten werden.

2. Zusammensetzung nach Anspruch 1, wobei die Zusammensetzung bei Raumtemperatur mindestens einen weiteren signifikanten Röntgenreflex in einem 2Theta-Bereich von > 7,5° bis ≤ 8,5° aufweist.

3. Zusammensetzung nach Anspruch 1, wobei die Zusammensetzung bei Raumtemperatur mindestens einen weiteren signifikanten Röntgenreflex in einem 2Theta-Bereich von > 6,2° bis ≤ 7,5° aufweist.

4. Zusammensetzung nach Anspruch 1 oder 2, wobei die teilkristalline Zusammensetzung C12 N-Methyl-N-Acylglucamin umfasst und der C-12 Acylglucamin-Anteil bezogen auf den gesamten N-Methyl-N-Acylglucamin-Gehalt größer oder gleich 25 mol-% und kleiner oder gleich 95 mol-% beträgt.

5. Zusammensetzung nach einem der vorhergehenden Ansprüchen, wobei die Zusammensetzung zusätzlich ein Lösemittel ausgewählt aus der Gruppe der ein-, zwei- oder dreiwertigen Alkohole umfasst.

6. Zusammensetzung nach einem der vorhergehenden Ansprüche, wobei die Konzentration der N-Alkyl-N-Acylglucamine in der Zusammensetzung größer oder gleich 5 Gewichts-% und kleiner oder gleich 65 Gewichts-% beträgt.

7. Zusammensetzung nach einem der vorhergehenden Ansprüche, wobei der Anteil mindestens eines N-Alkyl-N-Acylglucamins in der Zusammensetzung größer oder gleich 40 mol-% und kleiner oder gleich 95 mol-% bezogen auf den gesamten N-Alkyl-N-Acylglucamin-Gehalt beträgt.

8. Verfahren zur Herstellung einer teilkristallinen N-Alkyl-N-Acylglucamin-Zusammensetzung umfassend die Schritte:
a) Zur Reaktion bringen des N-Alkyl-Glucamins mit einer Base in wässriger Lösung,
b) Trocknen des Reaktionsproduktes aus Schritt a),
c) Vorlegen mindestens zweier Carbonsäurealkylester mit unterschiedlicher C6-C22-Kettenlänge,
d) Portionsweises Hinzufügen des unter b) erhaltenen, getrockneten Reaktionsproduktes zu der Mischung aus c) und
e) Ausreagieren der Zusammensetzung aus d) unter reduziertem Druck.

9. Verfahren nach Anspruch 9, wobei die Base aus Schritte a) Natronlauge ist.

10. Verfahren nach einem der Ansprüche 8 bis 9, wobei der Wassergehalt des getrockneten Reaktionsproduktes nach Schritt b) größer oder gleich 0,05 Gewichts-% und kleiner oder gleich 0,4 Gewichts-% beträgt.

11. Verfahren nach einem der Ansprüche 8 bis 10, wobei die Carbonsäurealkylester in Schritt c) in einem Lösemittel ausgewählt aus der Gruppe umfassend ein-, zwei- und/oder dreiwertige Alkohole vorgelegt werden.

12. Verfahren nach einem der Ansprüche 8 bis 11, wobei der Verfahrensschritt e) in einem Temperaturbereich von größer oder gleich 50 °C und kleiner oder gleich 175 °C durchgeführt wird.

13. Verfahren nach einem der Ansprüche 8 bis 12, wobei das Reaktionsprodukt aus Schritt e) in einem zusätzlichen Verfahrensschritt durch Zugabe mindestens eines protischen Lösemittels ausgewählt aus der Gruppe umfassend Wasser, ein-, zwei-, und/oder dreiwertige Alkohole oder Mischungen daraus, auf ein N-Alkyl-N-Acylglucamin-Gehalt von größer oder gleich 2,5 Gewichts-% und kleiner oder gleich 50 Gewichts-% eingestellt wird.

14. Verwendung einer teilkristallinen N-Alkyl-N-Acylglucamin-Zusammensetzung in Reinigungs-, kosmetischen, dermatologischen und/oder pharmazeutischen Anwendungen.

15. Verwendung nach Anspruch 14, wobei die Reinigungsanwendung eine Rinse-off Personal Care-Anwendung ist.
